# EUROPEAN PATENT APPLICATION

(11) **EP 3 954 453 A1**
(43) Date of publication of application: **16.02.2022**
(21) Application number: 21190652.4
(22) Date of filing: 10.08.2021
(51) Int. Cl.: B01D 39/08, B01D 39/16, B01D 46/00

(54) **ANTI-MICROBIAL, ANTI-ALLERGENIC BIO-FUNCTIONAL AIR FILTER MEDIUM FOR RESIDENTIAL AIR FILTRATION AND AIR FILTER ELEMENT**

(30) Priority: 13.08.2020 US 202016992663
(71) Applicant: MANN+HUMMEL LIFE SCIENCES & ENVIRONMENT HOLDING SINGAPORE PTE. LTD., Singapore 609930 (SG)
(72) Inventor: Tharval, Nilesh, Morrisville, 27560 (US); Keerl, David, 95447 Bayreuth (DE); Scope, Andreas, 09600 Oberschöna (DE); Schenk, Annette, Fuquay-Varina, 27526 (US)
(74) Representative: Mann + Hummel Intellectual Property

(57) **Abstract**

A bio-functional air filter medium is provided with a first filter layer that retains particles and at least one bio-functional carrier coated with or having embedded therein antimicrobial material and an anti-allergenic material. An air filter element having the bio-functional air filter medium is also disclosed. The bio-functional air filter medium is adapted to bind and capture allergens and block metabolism of bacteria while filtering air in high flow, low pressure drop residential or HVAC applications.

## Description

### TECHNICAL FIELD

The present invention relates to an anti-microbial, anti-allergenic bio-functional air filter medium, and replaceable air filter elements having the anti-microbial, anti-allergenic bio-functional air filter medium, particularly configured for use in high flow, low pressure drop residential air filtration or HVAC systems.

### BACKGROUND OF THE INVENTION

The field of the present disclosure generally relates to an anti-microbial, anti-allergenic bio-functional air filter medium, and air filter elements for high flow, low pressure drop residential applications and having an anti-microbial, anti-allergenic bio-functional air filter medium.

In some aspects of the invention, the bio-functional air filter medium is advantageously configured or packaged as an exchangeable air filter element particularly for residential air filtration. Further aspects of the invention find applicant in commercial building, or medical centers, hospitals, and may be configured for use in industrial or commercial building HVAC systems. The bio-functional air filter medium removes particulates, allergenic materials and microbial and bacterial matter from air within an interior building living spaces.

EP 1 882 511 A2 discloses, for example, a filter medium with bactericidal action, in particular for filtration of air for the passenger compartment of motor vehicles, comprised of at least one filter layer that filters out impurities and of a bactericidal filter layer downstream of said filter layer which is arranged at the clean side of the at least one filter layer and is spaced apart by a spacer layer from the at least one filter layer.

WO 2012168185 A1 describes a filter medium with antimicrobial action, in particular for filtration of air for the passenger compartment of motor vehicles, comprised of at least one first filter layer that filters out impurities and of a second filter layer neighboring said first filter layer. The second filter layer is attached to the inflow side of the first filter layer and contains antimicrobial materials.

US 10,300,419 B2 discloses, for example, a filter medium is provided with a first filter layer that retains particles and a second filter layer provided with an antimicrobial material and an anti-allergenic material. The anti-allergenic material contains polyphenols. The filter medium is designed particularly for filtering air for the passenger compartment of motor vehicles. A filter medium with bactericidal action, in particular for filtration of air for the passenger compartment of motor vehicles, comprised of at least one filter layer that filters out impurities and of a bactericidal filter layer downstream of said filter layer which is arranged at the clean side of the at least one filter layer and is spaced apart by a spacer layer from the at least one filter layer.

### SUMMARY OF THE INVENTION

In view of the above background, the present invention has the object to provide an improved bio-functional air filter medium, a bio-functional air filter and/or an improved bio-functional air filter arrangement.

The term, HVAC system, as used herein may be a system having at least one or more of the following capabilities: air heating, air cooling, humidity control, and air circulation.

Building for human occupation, such as residential homes, apartments, office buildings, hospitals, medical centers, schools, and retail stores, have one or more HVAC systems. Often the air from the interior space as well as outdoor air may be laden with undesired or harmful particulates, chemicals, odors, microbes, viruses, germs or other contaminants carried in the circulated air. As a matter of public health and safety, it is highly desirable to capture, disable or remove such pollutants.

Accordingly, the bio-functional air filter medium is a multi-layer filter medium having a first filter layer configured for capturing or removing particle impurities from air, and at least one bio-functional carrier layer adjacent to or lying against the first filter layer. The first filter layer and the at least one bio-functional carrier layer are arranged one after another in the air flow stream. The at least one bio-functional carrier layer includes anti-microbial, anti-bacterial and/or anti-allergenic materials, as discussed later below.

As used herein, we define term "bio-functional material" to include any of: antimicrobial materials which may include anti-viral materials, anti-bacterial materials, and anti-allergenic materials, which are arranged on, arranged in, or coated onto or embedded into the at least one bio-functional carrier layer.

As used herein, we define "at least one bio-functional carrier layer" to be one or a plurality of bio-functional carrier layers, arranged adjacently, each of which may include at least one of the bio-functional materials.

Advantageously, since the at least one bio-functional carrier layer includes the bio-functional material, the antimicrobial material can prevent metabolization or decomposition of the anti-allergenic material, in particular by fungi. This is particularly relevant for anti-allergenic materials in the form of polyphenols.

The at least one bio-functional carrier layer can comprise a support material, in particular a fiber material, for example, a nonwoven, laid fabric, woven fabric, felt, spunbond fabric, or a meltblown material, which comprises the bio-functional material or into which the antimicrobial materials, anti-bacterial materials, and anti-allergenic materials are introduced or incorporated (for example by being sprayed on). In some embodiments, individual layers of the at least one bio-functional carrier layer may be a single continuous layer of such a fiber material.

The bio-functional air filter medium is in particular configured for residential air filtration of air in homes, and finds advantageous use in heating, cooling or air purifying device applications. Advantageously, the comparatively high air permeability of the bio-functional air filter medium, which advantageously is preferably greater than or equal to 3000 l/m²/s, the bio-functional air filter medium is particularly well suited to residential home heating and cooling systems and air purifying devices and systems. Due to the high air permeability, the bio-functional air filter medium of the invention is particularly adaptable to application retrofitting an improved bio-functional air filter into existing heating, cooling systems. Such system typically have relatively weak air blowers designed for conventional filters and therefore cannot adapt to the higher pressure drops of other prior art filter solutions.

The antimicrobial, in particular antibacterial or biocidal, material protects the bio-functional air filter medium, or parts or layers thereof, from becoming infested with microorganisms such as fungi or fungal spores, in particular mold or mold spores, bacteria or algae which may be present in living, reproductive or proliferating form, or protects the bio-functional air filter medium from such microorganisms spreading within and from a penetrating growth of such microorganisms through the bio-functional air filter medium.

The anti-allergenic material can at least partially render innocuous for the human body, or its immune system, for example super-fine pollen particles and other allergenic substances that cannot always be retained completely by a filter medium. This leads to increased comfort within the building, room, home or other occupied filtered air space.

US 10,300,419 B2 discloses, for example, a filter medium provided with a first filter layer that retains particles and a second filter layer provided with an antimicrobial material and an anti-allergenic material. The disclosure of US 10,300,419 B2 is hereby incorporated by reference into this application, to the fullest extent permitted by the law.

As antimicrobial material, in particular zinc pyrithione can be used. Alternatively or additionally, octylisothiazolinone can be used as an antimicrobial material. The at least one second filter layer moreover may contain dimethyltetradecyl [3-(trimethoxysilyl)propyl ammonium chloride aka quaternary ammonia compound. The at least one second filter layer moreover may contain antimicrobial or anti-viral materials on the basis of nanosilver. The at least one second filter layer can also contain antimicrobial or anti-viral metals and metal compounds, in particular silver, copper, and aluminum compounds, and/or 2-bromo-2-nitropropane-1,3-diol, further isothiazolinone compounds, benzoic acid and its derivatives, benzalkonium halides, water-soluble coenzymes, oil-soluble coenzymes, plant extracts, antibiotics, biocidal metals, aliphatic and/or aromatic fatty acids and/or quaternary surfactants as antimicrobial materials. The application of a biocidal substance on air filtration applications is registered according to EU BPR 528/2012 and US PR 2000-1.

Advantageously, positively charged biocides binds to negatively charged cell membrane of microbes, advantageously penetrate the cell wall causing loss of cell wall integrity and ultimately loss of vital cell components of the microbe, and inhibiting growth.

As anti-allergenic materials, polyphenols such as catechines, tannins or flavonoids, are conceivable. In particular, caffeic acid, gallic acid, ellagic acid, tannic acid, cyanidin, procyanidin, proanthocyanidins, rutin, quercetin, resveratrol can be employed. Moreover, for example, tannin or tannic acid, in particular derived from wood (for example tree bark), apple extracts or citrus fruit extracts are conceivable. These material bind preferably allergenic substances so that the allergenic effect can be reduced or removed from the filtered air. Allergens are denatured by polyphenols, for example. The anti-allergenic material can moreover comprise anti-allergenic enzymes. Anti-allergenic enzymes cleave preferably allergenic proteins into smaller unharmful components. Generally speaking, the quantity of the polyphenols in the filter medium can be between 3 and 15 g/m², preferably between 5 and 10 g/m² (in particular in the at least one second filter layer). Polyphenols of natural origin are preferred as they have been shown to bind to and inactivate allergens.

In particular, the bifunctional air filter medium, preferably in the bio-functional carrier layer or layers, in at least in some embodiments, may contain one or several of the materials listed in the following Tables 1 and 2, even in any combination.

**Table 1**

| Material | Preferred quantity [g/m²] | More preferred quantity [g/m²] |
|---|---|---|
| tannin and/or tannic acid | 1 - 50 g/m² | 5 - 20 g/m² |
| dimethyltetradecyl[3 - (trimethoxysilyl)propyl] ammonium chloride | 0.5 - 30 g/m² | 1 - 15 g/m² |
| dye | 0.1 - 20 g/m² | 0.3 - 10 g/m² |
| surfactant | 0.05 - 5 g/m² | 0.1 - 3 g/m² |

**Table 2**

| Material | Preferred quantity [wt. %] | More preferred quantity [wt. %] |
|---|---|---|
| tannin and/or tannic acid | 0.05 - 80 % | 1 - 25 % |
| dimethyltetradecyl[3 - (trimethoxysilyl)propyl] ammonium chloride | 0.5 - 50 % | 1 - 20 % |
| dye | 0.1-30% | 0.3-10% |
| surfactant | 0.05 - 10 % | 0.1 - 3% |

The Tables 1 and 2 differ in their definition of the quantity of the materials introduced into the bio-functional carrier layer, i.e., on the one hand, according to weight per surface area and, on the other hand, weight percent (in each case referring to the bio-functional carrier layer).

We define antimicrobial material to be materials the prevent or inhibit the growth of microorganisms, for one example: bacteria, and may further have anti-viral effects. The antimicrobial material not only can be effective against bacteria but also can comprise a bacteriostatic and/or bactericidal as well as fungistatic and/or fungicidal action.

Bactericidal means an action that kills bacteria. In this context, the bacteria must be killed to a certain proportion, preferably at least 99% within the first 4 hours after its application. In comparison to this, bacteriostatic substances only have a growth-inhibiting action.

Quaternary ammonium compounds, being cationic surfactants, show affinity for bacteria and exhibit antibacterial efficacy under these conditions. It is believed that they interfere with the function of the cell membrane, resulting in leakage of cell components and eventually lysis, or destruction of the cell.

The antimicrobial material can include at least one active agent of the group comprising pyrithione and/or a metal salt thereof, wherein the metal salt is in particular alkali metal salt, in particular a sodium salt, an alkaline earth metal salt or a transition metal salt, preferably of the group zinc, manganese, copper, and iron, or a pyrithione derivative and/or a metal salt thereof, wherein the metal salt is in particular an alkali metal salt, in particular a sodium salt, an alkaline earth metal salt or a transition metal salt, preferably from the group zinc, manganese, copper, and iron. Alternatively or additionally, a quaternary ammonium salt of the general formula NR4+X- or R=NR2+X-, wherein R denotes herein an organic residue and wherein R can be the same or different, and wherein R is preferably at least one alkoxy group of the general formula -OCH3, a siloxy group of the general formula R3Si-O- or an alkoxysilyl group of the general formula R1R2R3Si-O-R4, in particular a trialkoxysilylpropyl group, and wherein X- is an anion, in particular a halide of the group F, CI, Br or I.

The pyrithione metal salt or pyrithione derivative metal salt can be a zinc salt, in particular zinc pyrithione.

Furthermore, it can be provided that the quaternary ammonium salt comprises a trialkoxysilylpropyl group, in particular is dimethyltetradecyl [3-(trimethoxsilyl)propyl] ammonium chloride or 3-(tri-methoxysilyl) propyldimethyl octadecyl ammonium chloride.

Preferably the biocidal material includes quaternary ammonium compounds which are cationic surfactants and further exhibit affinity for bacteria, and antibacterial efficacy.

Particularly preferable is dimethyltetradecyl [3-(trimethoxysilyl)propyl] ammonium chloride. Particulartly, dimethyltetradecyl [3-(trimethoxysilyl)propyl] ammonium chloride interferes with the function of the cell membrane, resulting in leakage of cell components out of the cell membrane and eventually lysis, or the destruction of the cell.

Quaternary ammonium compounds, being cationic surfactants, show affinity for bacteria and exhibit antibacterial efficacy under these conditions. It is believed that they interfere with the function of the cell membrane, resulting in leakage of cell components and eventually lysis, or destruction of the cell.

### Results from testing

The bio-functional filter medium itself is preferably of a multi-layer configuration. In the flow direction through the bio-functional filter medium, for example, the particle filter layer or layers, may be layered on abutting against an upstream side or a downstream side of the at least one bio-functional carrier layer.

In the bio-functional air filter medium having the particle retaining first filter layer and the at least one bio-functional carrier layer, according to the invention, the 2-step filtration and treatment of the air stream provides advantageously effective and efficient bio-functional filtration of the air flow.

In the first step, the bio-functional air filter medium separates pollen, allergens and bacteria, mold, fungi from the air flow through high efficiency fine dust filtration. Similar to in fine dust particle filtration, allergens are separated, particularly as allergens are typically 10-50 nm in size.

In the second step, the anti-allergenic material, ex: polyphenols, bind to allergens separated on filter medium, particularly in the at least one bio-functional carrier layer. The anti-allergenic material thereby inactivates allergens. The inactivated allergens lose their irritating effects (i.e. not recognized as allergens anymore).

Additionally, in the second step, bacteria and mold are captured and inhibited on the at least one bio-functional carrier layer. The microorganisms may remain on the bio-functional carrier layer, but are not viable anymore, thereby odors, bad smells etc. are reduced or eliminated.

According to another aspect of the invention, some embodiments may have a layer of the at least one bio-functional carrier layer having the antimicrobial material, and another layer of the at least one bio-functional carrier layer contains the anti-allergenic material.

According to a further embodiment, the first filter layer, and/or one or several of the at least one bio-functional carrier layer is embodied as a fine filter, in particular for filtering particles with a diameter of 10 µm and greater, preferably 2.5 µm and greater.

The allergen penetration, using grass pollen allergen, was tested by external institute Bifa Augsburg. The addition of the anti-microbial and anti-allergenic materials on the at least one bio-functional carrier layer of the invention resulted in an allergen penetration reduction of approximated 55% relative to the filter medium without the anti-microbial and anti-allergenic materials (tested in accordance with DIN 71460). The addition of the anti-microbial and anti-allergenic materials on the at least one bio-functional carrier layer of the invention resulted in an almost total inactivation (>95%) of separated allergens, test using grass pollen allergen, in comparison to filter medium without the anti-microbial and anti-allergenic materials. Allergens separation analyzed via ELISA, allergens separated in anti-allergen bio-functional carrier layer are all deactivated (ELISA tests -> no allergens detected due to inactivation), however allergens separated in reference or filtration layer (i.e. the first filter layer) were still active (ELISA tests -> >90% allergens were detected only in the first filter layer).

Regarding antimicrobial reduction, the addition of the anti-microbial and anti-allergenic materials on the at least one bio-functional carrier layer of the invention resulted in a greater than 95% separation of microorganisms in the bio-functional air filter medium (testing based on ISO 846 procedure). The reduction n in the testing was 99.98% for natural flora, 98% for curtobacterium and 100% for cladosporium.

Mechanical separation of microorganisms was fulfilled with every high efficient PM2.5 or better filter (>>90%). An analysis/investigation of what happens with captured microorganism (field samples) resulted in >98% reduction of antimicrobials due to at least one bio-functional carrier layer compared to non-treated filter.

According to the above and in this invention, the first filter layer and the at least one bio-functional carrier layer are preferably folded co-pleated (the layers are all corrugated together at the same time into pleats) for increasing the filtering surface area of the bio-functional air filter medium within a given filter space or filter medium edge boundary. In various configurations, the folded or corrugated filter medium can be provided at least on one side with a side strip of nonwoven or can be embedded in a cardboard or plastic frame. The folded or corrugated filter medium can be provided with a side strip, in particular a side strip of nonwoven, on at least one of its end face edges; these are the sides or end faces of the folded or corrugated bio-functional filter medium that exhibit a zigzag or corrugated shape.

In further configurations, the co-pleated bio-functional filter medium is arranged in a frame surrounding the peripheral edges of the folded or corrugated bio-functional filter medium and preferably having a grid or mesh portion of the frame extending across the inflow face and the outflow face of the co-pleated bio-functional filter medium, thereby providing support for the co-pleated bio-functional filter medium.

A primary object of the invention is provide a bio-functional air filter medium for residential air filtration. The bio-functional air filter medium having a first filter layer configured to retain particles; and a bio-functional carrier layer having a substrate comprising spunbond or meltblown fibers and having an air permeability greater than or equal to 3000 l/m²s. The bio-functional carrier layer includes an anti-microbial material which includes an anti-microbial material selected from the group consisting of: pyrithione, a pyrithione derivative, and/or a metal salt thereof. The bio-functional carrier layer further includes an anti-allergenic material selected from the group consisting of: catechines, tannin or tannic acid derived from wood, apple extracts or citrus bark, apple extracts or citrus fruit extracts, caffeic acid, gallic acid, tea extract, persimmon juice, ellagic acid, tannic acid, hydroxybenzoic acid compounds consisting of (2,5-dihydroxybenzoic acid, 2,6-dihydroxybenzoic acid, 2,4,6-trihydroxybenzoic acid), cyanidin, procyanidin, proanthocyanidins, rutin, quercetin, resveratrol, anti-allergenic enzymes or combinations thereof. The anti-allergenic material is adapted to bind to and capture allergenic substances in the bio-functional carrier layer so that an allergenic effect is reduced.

Preferably the anti-allergenic material and the anti-microbial material are applied onto the bio-functional carrier layer as a coating or introduced into the fibers of the bio-functional carrier layer, forming a positive-charged protective shield on the bio-functional carrier layer substrate, such that bacterial cells with their negative charge are attracted and inactivated when contacting the bio-functional carrier layer, blocking cell function and reproduction. The anti-microbial material is configured to block metabolism of bacteria and mold in order to avoid growth of these on the filter surface or decomposition of the anti-allergenic material in the bio-functional carrier layer.

In another aspect of the invention, the metal salt is an alkali metal salt, a sodium salt, an alkaline earth metal salt or a transition metal salt, of the group zinc, manganese, copper, and iron, a pyrithione derivative and/or a metal salt thereof, wherein the metal salt is of the group consisting of: zinc, manganese, copper, and iron, a pyrithione derivative and/or a metal salt thereof, and/or a quaternary ammonium salt of the general formula NR4+X- or R=NR2+X-, wherein R is an organic residue, wherein R is the same or different, and wherein R is at least one alkoxy group of the general formula -OCH3, a siloxy group of the general formula R3Si-O- or an alkoxysilyl group of the general formula R1 R2R3Si-O-R4.

In another aspect of the invention, the alkoxysilyl group can be a trialkoxysilylpropyl group.

In another aspect of the invention, the pyrithione metal salt may be zinc pyrithione.

In another aspect of the invention, the quaternary ammonium salt may include a trialkoxysilylpropyl group selected from the group consisting of: dimethyltetradecyl [3-(trimethoxsilyl)propyl] ammonium chloride or 3-(trimethoxysilyl) propyldimethyl octadecyl ammonium chloride.

In another aspect of the invention, the anti-microbial material further includes an anti-microbial material selected from the group consisting of: quaternary ammonium compounds, octylisothiazolinone, silver, copper, aluminum compounds, 2-bromo-2-nitropropane-1,3-diol, isothiazolinone compounds, benzoic acid and its derivatives, benzalkonium halides, water-soluble coenzymes, oil-soluble coenzymes, plant extracts, antibiotics, biocidal metals, aliphatic and/or aromatic fatty acids, or combinations thereof.

In another aspect of the invention, the first filter layer and the bio-functional carrier layer are preferably overlaid against each other and co-pleated together, forming a pleated bio-functional air filter medium.

In another aspect of the invention, the quantity of the anti-microbial material in the bio-functional carrier layer is between 3 and 15 g/m².

In another aspect of the invention, the fibers of the bio-functional carrier layer include coarse fibers having a diameter in the range of 15-50 µm.

In yet another aspect of the invention, an anti-microbial and anti-allergenic bio-functional exchangeable air filter element is provided, including the above discussed bio-functional air filter medium, wherein the first filter layer and the bio-functional carrier layer are overlaid against each other and co-pleated together, forming a pleated bio-functional air filter medium. A frame is arranged on and extending fully across an inlet flow face and an outlet flow face of the bio-functional air filter medium, the frame enclosing and supporting the bio-functional air filter medium, forming a unitary replaceable anti-microbial and anti-allergenic bio-functional air filter element.

In another aspect of the invention, the quaternary ammonium salt includes a trialkoxysilylpropyl group selected from the group consisting of: dimethyltetradecyl [3-(trimethoxsilyl)propyl] ammonium chloride or 3-(trimethoxysilyl) propyldimethyl octadecyl ammonium chloride.

In another aspect of the invention, wherein the quantity of the polyphenols in the bio-functional filter layer is between 3 and 15 g/m².

Further possible implementations of the invention comprise also combinations, not explicitly mentioned, of features or method steps disclosed above or in the following in regard to the embodiments. In this context, a person of skill in the art will also add individual aspects as improvements or supplements to the respective basic form of the bio-functional air filter medium and of the bio-functional air filter element.

Further configurations of the invention are subject matter of the dependent claims as well as of the embodiments of the invention described in the following. In the following, the invention will be explained in more detail with the aid of embodiments with reference to the attached figures.

### BRIEF DESCRIPTION OF THE DRAWINGS

The accompanying Figures, where like reference numerals refer to identical or functionally similar elements throughout the separate views and which together with the detailed description below are incorporated in and form part of the specification, serve to further illustrate various embodiments and to explain various principles and advantages all in accordance with the present invention.

Features of the present invention, which are believed to be novel, are set forth in the drawings and more particularly in the appended claims. The invention, together with the further objects and advantages thereof, may be best understood with reference to the following description, taken in conjunction with the accompanying drawings. The drawings show a form of the invention that is presently preferred; however, the invention is not limited to the precise arrangement shown in the drawings, but is to be further understood from the disclosure of the specification and the claims.
Fig. 1 shows a schematic illustration of the bio-functional filter medium having functional layers which are co-pleated together to form a folded, pleated the bio-functional filter medium, consistent with the present inventive disclosure; and
Fig. 2 shows a schematic perspective illustration of the bio-functional filter medium, as in Fig 1, arranged within a supporting frame and packed for replaceable installation.

Skilled artisans will appreciate that elements in the figures are illustrated for simplicity and clarity and have not necessarily been drawn to scale. For example, the dimensions of some of the elements in the figures may be exaggerated relative to other elements to help to improve understanding of embodiments of the present invention.

### DETAILED DESCRIPTION

In this document, relational terms such as first and second, top and bottom, and the like may be used solely to distinguish one entity or action from another entity or action without necessarily requiring or implying any actual such relationship or order between such entities or actions. The terms "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus. An element proceeded by "comprises ... a" does not, without more constraints, preclude the existence of additional identical elements in the process, method, article, or apparatus that comprises the element.

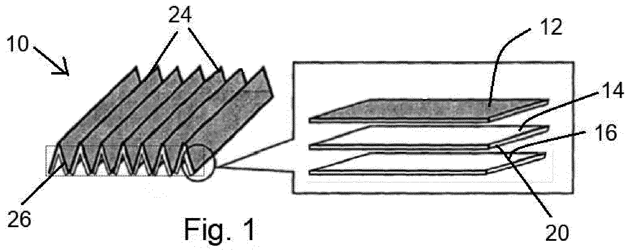

Figure 1 is a schematic illustration of the bio-functional filter medium according to this inventive disclosure, and configured with anti-microbial and anti-allergenic materials as discussed and presented in the Summary of the Invention section.

The bio-functional air filter medium 10 includes HVAC system applications, having a first filter layer 12 configured to retain particles and dust; and at least one bio-functional carrier layer 14 arranged at or against a flow face of the first filter layer 12. The at least one bio-functional carrier layer 14 having a substrate 20 which preferably includes spunbond or meltblown fibers and having an air permeability greater than or equal to 3000 l/m²s. Preferably the substrate 20 of the at least one bio-functional carrier layer 14 is comprises or consisting of coarse fibers having a diameter in the range of 15-50 µm.

It is advantageous if the bio-functional air filter medium 10 has an air permeability greater than or equal to 3000 l/m²s. The first filter layer 12 and the bio-functional carrier layer14 are overlaid against each other and co-pleated together (as in Fig. 1), forming a pleated bio-functional air filter medium 20.

Consistent with the invention, the at least one bio-functional carrier layer 14 includes one or more anti-microbial materials which includes an anti-microbial material selected from the group consisting of: pyrithione, a pyrithione derivative, and/or a metal salt thereof, or other anti-microbial materials presented and discussed in detail in the Summary of the Invention section.

Consistent with the invention, the at least one bio-functional carrier layer 14 further includes one or more anti-allergenic materials selected from the group consisting of: catechines, tannin or tannic acid derived from wood, apple extracts or citrus bark, apple extracts or citrus fruit extracts, caffeic acid, gallic acid, tea extract, persimmon juice, ellagic acid, tannic acid, hydroxybenzoic acid compounds consisting of (2,5-dihydroxybenzoic acid, 2,6-dihydroxybenzoic acid, 2,4,6-trihydroxybenzoic acid), cyanidin, procyanidin, proanthocyanidins, rutin, quercetin, resveratrol, anti-allergenic enzymes or combinations thereof and may include the anti-allergenic materials discussed in detail in the Summary of the Invention section. The anti-allergenic material advantageously binds to and captures allergenic substances in the bio-functional carrier layer so that an allergenic effect is reduced. Preferably, a quantity of polyphenols in the at least one bio-functional filter layer 14 is between 3 and 15 g/m².

The anti-allergenic material and the anti-microbial material are applied onto the at least one bio-functional carrier layer 14 as a coating or introduced into the fibers of the bio-functional carrier layer 14, forming a positive-charged protective shield on the bio-functional carrier layer substrate 20, such that bacterial cells with their negative charge are attracted and inactivated when contacting the bio-functional carrier layer 14, blocking cell function and reproduction.

The anti-microbial material is configured to block metabolism of bacteria and mold in order to avoid growth of these on the bio-functional carrier layer 14 substrate 20 or filter surface and prevent or decomposition of the anti-allergenic material in the at least one bio-functional carrier layer 14.

When the at least one bio-functional carrier layer 14 is embodied as two or more bio-functional carrier layers 14, 16, the anti-allergenic material may possibly be coated onto, embedded on or into a first one of bio-functional carrier layers 14 and the anti-microbial material may possibly be coated onto, embedded on or into a second one of bio-functional carrier layers 16.

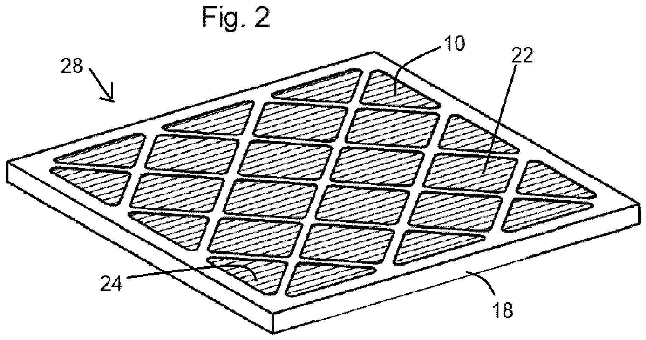

Fig. 2 shows a schematic perspective illustration of the co-pleated bio-functional filter medium 22, as in Fig 1, arranged within a supporting frame 18 and packaged for replaceable installation into an air cleaning device, or HVAC system or air handling system.

Referring to Fig 1 with Fig 2, the bio-functional air filter medium 10 may have the first filter layer 12 arranged upstream of the at least one bio-functional layer 14, the first filter layer 12 forming an inlet flow face of the bio-functional air filter medium 10. In Fig 1, the at least one bio-functional carrier layer 14 is arranged downstream of the first filter layer 12 or particle filter layer 12 and may form an outlet flow face of the bio-functional air filter medium 10.

To minimize pressure drop and provide for a high air permeability of greater than or equal to 3000 l/m²s, it is preferred that the first filtration or particle layer is overlaid onto the bio-functional carrier layer 14 without adhesives between the layers to bind the layers together. Preferably the layers are loosely arranged on each other and then co-pleated together to form the pleated bio-functional air filter medium 22 having folds or pleats 22 and pleat end edges 26.

The supporting frame 18, when included, preferably fully surrounds the pleated bio-functional air filter medium 22, enclosing the pleat end edges 26 at lateral sides of the support frame and functional retaining the loosely overlaid filter layer 12 and the at least one bio-functional carrier layer 14 in position and alignment within the support frame 18.

Other chemically related inventive aspects of the at least one bio-functional carrier layer 14 are discussed and presented more fully in the Summary of the Invention section and in the originally presented claims.

In the foregoing specification, specific embodiments of the present invention have been described. However, one of ordinary skill in the art appreciates that various modifications and changes can be made without departing from the scope of the present invention as set forth in the claims below. Accordingly, the specification and figures are to be regarded in an illustrative rather than a restrictive sense, and all such modifications are intended to be included within the scope of the present invention. The benefits, advantages, solutions to problems, and any element(s) that may cause any benefit, advantage, or solution to occur or become more pronounced are not to be construed as a critical, required, or essential features or elements of any or all the claims. The invention is defined solely by the appended claims including any amendments made during the pendency of this application and all equivalents of those claims as issued.

## Claims

1. A bio-functional air filter medium (10, 22) for residential air filtration, comprising:
a first filter layer (12) configured to retain particles; and
a bio-functional carrier layer (14, 16) having a substrate (20) comprising spunbond or meltblown fibers and having an air permeability greater than or equal to 3000 l/m²s;
wherein the bio-functional carrier layer (14, 16) further comprises:
an anti-microbial material which includes an anti-microbial material selected from the group consisting of: pyrithione, a pyrithione derivative, and/or a metal salt thereof; and
an anti-allergenic material selected from the group consisting of: catechines, tannin or tannic acid derived from wood, apple extracts or citrus bark, apple extracts or citrus fruit extracts, caffeic acid, gallic acid, tea extract, persimmon juice, ellagic acid, tannic acid, hydroxybenzoic acid compounds consisting of (2,5-dihydroxybenzoic acid, 2,6-dihydroxybenzoic acid, 2,4,6-trihydroxybenzoic acid), cyanidin, procyanidin, proanthocyanidins, rutin, quercetin, resveratrol, anti-allergenic enzymes or combinations thereof;
wherein the anti-allergenic material binds to and captures allergenic substances in the bio-functional carrier layer so that an allergenic effect is reduced;
wherein the anti-allergenic material and the anti-microbial material are applied onto the bio-functional carrier layer (14, 16) as a coating or introduced into the fibers of the bio-functional carrier layer (14, 16), forming a positive-charged protective shield on the bio-functional carrier layer substrate (20), such that bacterial cells with their negative charge are attracted and inactivated when contacting the bio-functional carrier layer (14, 16), blocking cell function and reproduction;
wherein the anti-microbial material is configured to block metabolism of bacteria and mold in order to avoid growth of these on the filter surface or decomposition of the anti-allergenic material in the bio-functional carrier layer (14, 16).

2. The bio-functional air filter medium (10, 22) according to claim 1, wherein
the metal salt is an alkali metal salt, a sodium salt, an alkaline earth metal salt or a transition metal salt, of the group zinc, manganese, copper, and iron, a pyrithione derivative and/or a metal salt thereof,
wherein the metal salt is of the group consisting of: zinc, manganese, copper, and iron, a pyrithione derivative and/or a metal salt thereof, and/or a quaternary ammonium salt of the general formula NR₄⁺X⁻ or R=NR₂⁺X⁻, wherein R is an organic residue, wherein R is the same or different, and wherein R is at least one alkoxy group of the general formula -OCH₃, a siloxy group of the general formula R³Si-O- or an alkoxysilyl group of the general formula R¹R²R³Si-O-R⁴.

3. The bio-functional air filter medium (10, 22) according to claim 2, wherein
the alkoxysilyl group is a trialkoxysilylpropyl group.

4. The bio-functional air filter medium (10, 22) according to claim 2, wherein
the pyrithione metal salt is zinc pyrithione.

5. The bio-functional air filter medium (10, 22) according to claim 2, wherein
the quaternary ammonium salt comprises a trialkoxysilylpropyl group selected from the group consisting of: dimethyltetradecyl [3-(trimethoxsilyl)propyl] ammonium chloride or 3-(tri-methoxysilyl) propyldimethyl octadecyl ammonium chloride.

6. The bio-functional air filter medium (10, 22) according to claim 1, wherein
the anti-microbial material further includes an anti-microbial material selected from the group consisting of: quaternary ammonium compounds, octylisothiazolinone, silver, copper, aluminum compounds, 2-bromo-2-nitropropane-1,3-diol, isothiazolinone compounds, benzoic acid and its derivatives, benzalkonium halides, water-soluble coenzymes, oil-soluble coenzymes, plant extracts, antibiotics, biocidal metals, aliphatic and/or aromatic fatty acids, or combinations thereof.

7. The bio-functional air filter medium (10, 22) according to claim 1, wherein
the first filter layer (12) and the bio-functional carrier layer (14, 16) are overlaid against each other and co-pleated together, forming a pleated bio-functional air filter medium (22).

8. The bio-functional air filter medium (10, 22) according to claim 7, wherein
the first filter layer (12) is arranged upstream of the bio-functional carrier layer (14, 16), the first filter layer (12) forming an inlet flow face of the bio-functional air filter medium (10, 22);
the bio-functional carrier layer (14, 16) is arranged downstream of the first filter layer (12) and forms an outlet flow face of the bio-functional air filter medium (10, 22).

9. The bio-functional air filter medium (10, 22) according to claim 1, wherein
a quantity of the anti-microbial material in the bio-functional carrier layer (14, 16) is between 3 and 15 g/m².

10. The bio-functional air filter medium (10, 22) according to claim 1, wherein
the fibers of the bio-functional carrier layer (14, 16) comprise coarse fibers having a diameter in the range of 15-50 µm.

11. An anti-microbial and anti-allergenic bio-functional air filter element for residential air filtration and HVAC systems, comprising:
the bio-functional air filter medium (10, 22) according to claim 2;
wherein the first filter layer (12) and the bio-functional carrier layer (14, 16) are overlaid against each other and co-pleated together, forming a pleated bio-functional air filter medium (22);
a frame (18) arranged on and extending fully across an inlet flow face and an outlet flow face of the bio-functional air filter medium, the frame enclosing and supporting the bio-functional air filter medium (10, 22), forming a unitary replaceable anti-microbial and anti-allergenic bio-functional air filter element.

12. The anti-microbial and anti-allergenic bio-functional air filter element according to claim 11, wherein
the quaternary ammonium salt comprises a trialkoxysilylpropyl group selected from the group consisting of: dimethyltetradecyl [3-(trimethoxsilyl)propyl] ammonium chloride or 3-(tri-methoxysilyl) propyldimethyl octadecyl ammonium chloride.

13. The anti-microbial and anti-allergenic bio-functional air filter element according to claim 10, wherein
a quantity of the polyphenols in the bio-functional filter layer is between 3 and 15 g/m².
